(19) Europäisches Patentamt
European Patent Office
Office européen des brevets

(11) Veröffentlichungsnummer: **0 079 432**
**B1**

(12) # EUROPÄISCHE PATENTSCHRIFT

(45) Veröffentlichungstag der Patentschrift: **24.10.84**

(51) Int. Cl.³: **C 07 C 57/04,** C 07 C 51/00

(21) Anmeldenummer: **82107737.7**

(22) Anmeldetag: **24.08.82**

(54) **Verfahren zur Herstellung von Methacrylsäure aus Isobutyraldehyd.**

(30) Priorität: **27.10.81 DE 3142487**

(43) Veröffentlichungstag der Anmeldung: **25.05.83 Patentblatt 83/21**

(45) Bekanntmachung des Hinweises auf die Patenterteilung: **24.10.84 Patentblatt 84/43**

(84) Benannte Vertragsstaaten: **AT BE CH DE FR GB IT LI NL**

(56) Entgegenhaltungen:
**DE - A - 1 768 253**
**DE - A - 3 001 911**
**DE - C - 2 129 920**

(73) Patentinhaber: **CHEMISCHE WERKE HÜLS AG, - RSP Patente / PB 15 - Postfach 13 20, D-4370 Marl 1 (DE)**

(72) Erfinder: **Brockhaus, Rudolf, Dr., Holsteiner Strasse 19, D-4370 Marl (DE)**
Erfinder: **Franke, Hans-Jürgen, Freiheitstrasse 13, D-4270 Dorsten (DE)**

**EP 0 079 432 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Methacrylsäure und deren Ester sind wertvolle Polymerisationsrohstoffe. Die Säure oder deren Ester werden in der Regel aus Aceton und Cyanwasserstoff nach dem sogenannten Cyanhydrinprozess hergestellt. Das Acetoncyanhydrin setzt man in konzentrierter Schwefelsäure zu Methacrylamidsulfat um. Die weitere Umsetzung zur Methacrylsäure oder deren Ester ist ebenfalls allgemein geübte technische Praxis („Stanford Research Institute Report", Bd. 11, S. 27 f.). Zusammen mit der Methacrylsäure oder deren Ester fällt zwangsweise Ammonsulfat an. Dieses ist ein als Dünger in begrenztem Mass absetzbares lästiges Koppelprodukt. Ausser dem Anfall dieses Nebenproduktes hat dieses Verfahren den Nachteil, dass man in der ersten Stufe mit dem giftigen Cyanwasserstoff arbeiten muss. Dies ist der Anlass für zahlreiche Bemühungen um andere Synthesewege.

So hat man versucht, Isobutylen über Methacrolein zu Methacrylsäure zu oxidieren. Die bisherigen Ausbeuten und der technische Aufwand dieses zweistufigen Gasphasenoxidationsprozesses („SRI Report", Bd. 11, S. 35 bis 37) reichen noch nicht für eine allgemeine grosstechnische Anwendung dieses Prozesses auf Basis Isobuten.

Es wurde weiter vorgeschlagen, aus Isobuten zunächst t-Butanol herzustellen, diesen Alkohol in der Gasphase zu Methacrolein umzusetzen und dieses in Methacrylsäure zu überführen („Hydrocarbon Processing", Februar 1979, S. 105 bis 107). Auch dieser Prozess konnte den Cyanhydrinprozess bisher nicht ersetzen.

Schliesslich sind („Stanford Research Institut Report", Bd. 11, S. 30) noch mehrere Verfahren bekannt, Isobuten mit Salpetersäure oder einem Gemisch von Salpetersäure und Stickstoffdioxid oder deren Essigsäuregemische zu α-Hydroxyisobuttersäure, einem Vorprodukt für Methacrylsäure, zu oxidieren. Aus der α-Hydroxyisobuttersäure erhält man durch Wasserabspaltung Methacrylsäure (DE-PS Nr. 1568948 = GB-PS Nr. 1080473 und CA-PS Nr. 771714, DE-OS Nr. 1768253 = GB-PS Nr. 1179987).

Diese Methoden bringen zwar teilweise ganz gute Ausbeuten auf Buten bezogen, aber die Reaktionslösungen und Zwischenprodukte sind explosiv. Ausserdem wird die Stickstoff/Sauerstoffverbindung bis zu $N_2$ oder $N_2O$ und nicht zu NO reduziert. Nur NO kann aber im Gegensatz zu $N_2$ oder $N_2O$ mit Luftsauerstoff wieder reoxidiert und als Salpetersäure recycliert werden.

Man hat bisher auch versucht, Isobuttersäure zu Methacrylsäure zu dehydrieren (DE-PS Nr. 2129920 = GB-PS Nr. 1332558, DE-PS Nr. 2208580 = GB-PS Nr. 1360550). Auch auf diesem Weg konnten bisher keine technisch befriedigenden Ergebnisse erzielt werden.

Es besteht daher ein grosses Interesse an einem verbesserten Verfahren, Methacrylsäure aus leicht zugänglichen Einsatzstoffen auf technisch einfachem Wege in guter Ausbeute herzustellen.

Die sich hieraus ergebende Aufgabe wird erfindungsgemäss entsprechend den Angaben der Patentansprüche gelöst.

Die Stufen 3 und 5 des erfindungsgemässen Verfahrens sind neu.

Das nachfolgende Formelschema erläutert das Verfahren

1. 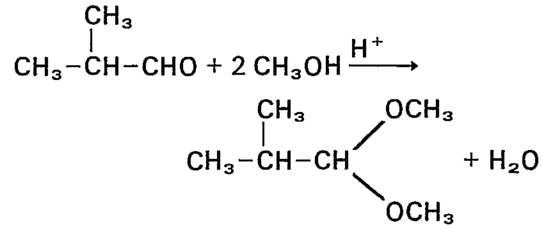

Isobutyraldehyd + 2 Methanol → Acetal + Wasser

2. 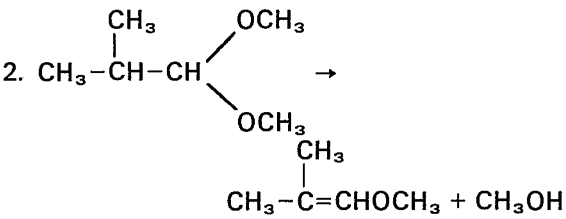

Acetal → Isobutenylether + Methanol
(Acetalspaltung)

3. 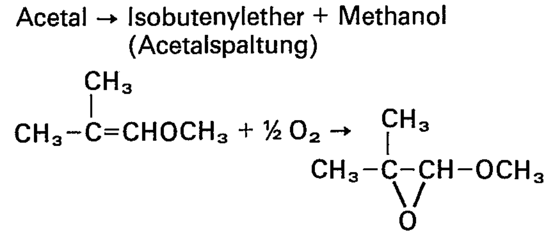

Oxidation von Isobutenylether mit molekularem Sauerstoff zum Epoxid (Isobutenyloxidmethylether)

4. 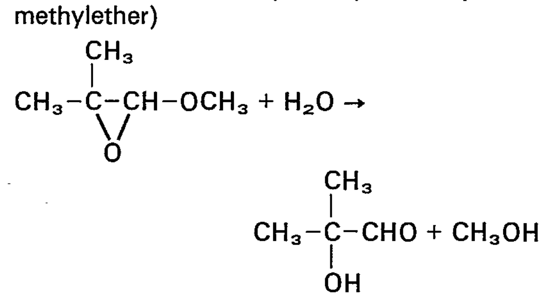

Wasseranlagerung an das Epoxid unter Bildung von α-Hydroxyisobutyraldehyd und Methanol

5. 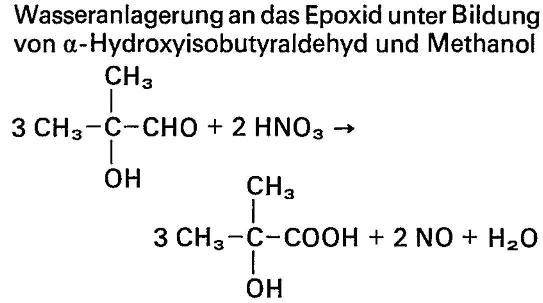

Oxidation von α-Hydroxyisobutyraldehyd mit Salpetersäure zu α-Hydroxyisobuttersäure

6. 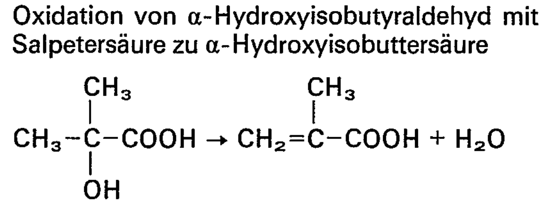

Wasserabspaltung aus α-Hydroxyisobuttersäure zu Methacrylsäure

Die Stufe 3, die Oxidation des ungesättigten Ethers mit molekularem Sauerstoff oder einem sauerstoffhaltigen Gasgemisch wie Luft in Gegenwart einer Lauge bei Temperaturen von 30 bis 70° C ist neu und unerwartet.

Bisher war für die Epoxidierung der wesentlich aufwendigere Einsatz von Peroxiden erforderlich. Somit bringt diese Stufe einen überraschenden technischen Fortschritt. Die Selektivität ist wie allgemein bei Oxidationen eine Funktion des Umsatzes und nimmt mit steigendem Umsatz ab. Als Nebenprodukte fallen geringe Mengen an Aceton und Alkylformiat sowie α-Hydroxyisobutyraldehydacetal an.

Aceton ist ein brauchbares Nebenprodukt, das in einer Menge von 10 bis 20%, bezogen auf das Epoxid, anfällt. Das Acetal wird in den weiteren Verfahrensstufen mitverarbeitet. CO und $CO_2$ fallen bei der Oxidation praktisch nicht an. Die zugesetzte Lauge ist billig. Sie stabilisiert gleichzeitig das gebildete Epoxid.

Bei der Salpetersäureoxidation in Stufe 5 enthalten die nach der Oxidation entweichenden Gase überraschenderweise im Gegensatz zu den Verfahren des Standes der Technik („SRI-Report", lit. cit.) im wesentlichen nur die Stickoxide NO und $NO_2$ sowie bei Vorhandensein von Methanolresten Ester, insbesondere Salpetrigsäureester. $N_2$ und $N_2O$ entstehen nur in geringen Mengen. Dies ist ein erheblicher Vorteil gegenüber diesen bisherigen Verfahren, weil damit die Salpetersäure als regenerierbarer Sauerstoffüberträger dient. Als Salpetersäure kann handelsübliche, konzentrierte oder rauchende Säure eingesetzt werden.

Die Acetalbildung (Stufe 1) erfolgt durch Umsetzung von Isobutyraldehyd mit einem Alkohol, bevorzugt Methanol, im allgemeinen in etwa stöchiometrischen Mengen. Die Acetalbildung verläuft wie bekannt in Gegenwart von sauren Katalysatoren, z.B. 0,01 bis 0,15 Gew.-% p-Toluolsulfonsäure oder Schwefelsäure bezogen auf die Einsatzmenge bzw. an Ionenaustauschern bei Temperaturen von 60 bis 100° C.

Im allgemeinen gibt man den Isobutyraldehyd bei Temperaturen von 10 bis 30° C mit dem Alkohol zusammen und erwärmt die Mischung anschliessend auf 60 bis 100° C. Das Gleichgewicht liegt bei einem Umsatz von 60 bis 70% und stellt sich in wenigen Minuten ein. Anschliessend neutralisiert man den sauren Katalysator mit der berechneten Menge einer Lauge, beispielsweise NaOH bzw. man entfernt den Ionenaustauscher. Zur Abtrennung des nichtumgesetzten Alkohols und Isobutyraldehyds wäscht man beispielsweise das Reaktionsprodukt mehrmals mit Wasser. Das gewaschene Produkt wird destilliert. Das Waschwasser destilliert man zur Rückgewinnung von Isobutyraldehyd und Alkohol, beispielsweise Methanol an.

Die Spaltung des erhaltenen Acetals zum Isobutenylether (Stufe 2) erfolgt direkt oder in einem inerten Verdünnungsmittel wie Paraffinöl in Gegenwart eines sauren Katalysators, beispielsweise p-Toluolsulfonsäure, bei Temperaturen von 80 bis 150° C. Man destilliert das Gemisch von Ether und Alkohol, vorzugsweise Methanol, während der Spaltung laufend ab. Man befreit den Ether durch Wasserwäsche vom Alkohol, trocknet gegebenenfalls und destilliert. Den Alkohol gewinnt man aus dem Wasser durch Andestillieren zurück.

Die Ausbeuten der ersten beiden Schritte hängen von der Intensität der Alkohol-, insbesondere Methanol- und Isobutyraldehydrückgewinnung aus dem Wasser ab und betragen 90 bis 95%. Ohne Wasseraufbereitung erreicht man eine Ausbeute von ca. 40%.

Die Oxidation des Isobutenylethers (Stufe 3) zum Isobutenyloxidether erfolgt bevorzugt in einer Oxidationsapparatur für Flüssigphasenoxidationen, z.B. einem Blasensäulenreaktor. Man begast den Isobutenylether in Gegenwart von 50 bis 500 ppm einer Lauge bei Temperaturen von 30 bis 70, vorzugsweise von 40 bis 50° C, mit molekularem Sauerstoff. Grössere Mengen Lauge bringen keine Verbesserung. Als Lauge eignen sich Hydroxyde der Alkali- und Erdalkalimetalle in alkoholischer oder auch wässeriger Lösung, vorzugsweise KOH und NaOH. Als molekularen Sauerstoff setzt man reinen molekularen Sauerstoff oder ein sauerstoffhaltiges Gasgemisch, vorzugsweise Luft, ein. Den Sauerstoff gibt man bevorzugt feinverteilt in die Reaktion, beispielsweise über eine Fritte. Das Abgas aus der Oxidation enthält nichtumgesetzten Sauerstoff, bzw. nichtumgesetztes sauerstoffhaltiges Gasgemisch, aber praktisch weder CO noch $CO_2$. Mit dem Abgas ausgetragene Leichtsieder, wie z.B. Methanol, Aceton und Alkylformiat, lassen sich in einem Kühlsystem abscheiden. Es ist vorteilhaft, die Oxidation bei einer Temperatur von 30 bis 50° C mit einer grösseren Menge von 15 bis 20 l molekularem Sauerstoff/kg Isobutenylether zu beginnen und in laufender Anpassung an die umsatzabhängige Reaktionsgeschwindigkeit nach einem Umsatz von 80 bis 90% bei einer Temperatur von 55 bis 70, vorzugsweise bis 60° C, und einer Sauerstoffmenge von 5 bis 10 l/kg Isobutenylether zu Ende zu führen. Man erreicht hiermit einen fast vollständigen Sauerstoffumsatz. Weitere Temperaturerhöhung verringert die Selektivität der Reaktion und bewirkt Produktaustrag. Der Produktaustrag lässt sich durch Arbeiten unter Druck verringern, erfordert jedoch technischen Aufwand. Man kann das rohe Epoxid direkt weiterverarbeiten, man kann es jedoch auch zuvor destillativ reinigen. Zwecks Neutralisation evtl. entstandener organischer Säuren und Epoxidstabilisierung versetzt man den Reaktoraustrag mit 0,5 bis 2,5 Gew.-% einer Lauge, vorzugsweise KOH oder NaOH in wenig Methanol gelöst, und fraktioniert bei Unterdruck. Zunächst destilliert man vorzugsweise bei einem Druck von 250 bis 300 bar den nichtumgesetzten Ether ab. Die Destillation des Epoxides erfolgt vorzugsweise bei einem Druck von 1 bis 30 mbar. Die Reinheit beträgt 95 bis 99% Epoxid.

Zur Hydrolyse (Stufe 4) setzt man das reine Epoxid oder das rohe Epoxid gemeinsam mit dem danach überdestillierenden α-Hydroxyisobutyraldehydacetal ein. Zur Hydrolyse legt man Wasser in

einer Menge von 1,1 bis 1,8 mol/mol Epoxid vor und setzt das Epoxid bei einer Temperatur von 60 bis 66° C unter Rühren langsam zu. Die Reaktionstemperatur wird durch Kühlen bzw. durch Sieden des bei der Reaktion anfallenden Alkohols gehalten. Nach der Umsetzung ist es zweckmässig, aber nicht verfahrensnotwendig, die Hauptmenge des Alkohols, beispielsweise Methanol, durch vorsichtiges Andestillieren – evtl. zur Hydrolyse von Acetal in schwach saurem Medium – zurückzugewinnen. Zurückbleibender Alkohol bildet in der nachfolgenden Oxidationsstufe Ester, insbesondere mit salpetriger Säure. Die optimale Wassermenge hängt ab vom gewünschten Grad der Hydrolyse einerseits und der die nachfolgende $HNO_3$-Oxidation beeinträchtigenden Verdünnung andererseits.

Die anschliessende Oxidation des α-Hydroxyisobutyraldehyds (Stufe 5) erfolgt nach der Hydrolyse direkt oder mit dem verbleibendem Rückstand nach dem Abdestillieren des Alkohols. Man oxidiert mit rauchender oder konzentrierter Salpetersäure bei Temperaturen von 20 bis 110° C mit einer Menge von 1,2 bis 2 mol Salpetersäure pro Mol Aldehyd, vorzugsweise ohne Katalysatorzusatz. Der Zusatz von üblichen Salpetersäureoxidationskatalysatoren wie Cersalzen oder Vanadiumverbindungen ist möglich, aber nicht erforderlich. Im Rohepoxid enthaltene Nebenprodukte, wie z.B. α-Hydroxyisobutyraldehyddimethylacetal werden zum gewünschten Endprodukt oxidiert. Man legt die Salpetersäure oder den α-Hydroxyisobutyraldehyd vor. Eine geringere Konzentration kann man durch eine höhere Reaktionstemperatur ausgleichen. Bei den Stufen 4 und 5 erreicht man insgesamt Ausbeuten $\geqslant$ 95 Mol-%, bezogen auf die Stufen Epoxid-Hydrolyse bis Oxidation.

Das Rohprodukt aus dieser Oxidationsstufe fraktioniert man bevorzugt bei 1 bis 200 mbar Unterdruck. Nach einem Vorlauf, der im wesentlichen aus Wasser und Salpetersäure besteht, erhält man die α-Hydroxyisobuttersäure rein weiss und kristallin. Sie hat einen Schmelzpunkt von $\geqslant$ 75° C. Essigsäure fällt praktisch nicht an. Methacrylsäure entsteht in dieser Stufe noch nicht.

Die Wasserabspaltung aus der α-Hydroxyisobuttersäure zur Methacrylsäure (Stufe 6) erfolgt in bekannter Weise, beispielsweise nach den Angaben der DE-PS Nr. 1568948 = GB-PS Nr. 1080473 und CA-PS Nr. 771714 und der DE-OS Nr. 1768253 = GB-PS Nr. 1179987.

Man erhitzt beispielsweise die α-Hydroxyisobuttersäure in Gegenwart ihrer Metallsalze, vorzugsweise der Salze der Alkali- und Erdalkalimetalle bei Atmosphärendruck auf ca. 200° C in einem Destillationskolben mit aufgesetzter Kolonne. Unter diesen Bedingungen kommt es zur Dehydratisierung der α-Hydroxyisobuttersäure. Die Reaktionsprodukte, die bei 93 bis 157° C überdestillieren, enthalten Methacrylsäure in einer Ausbeute von etwa 95%. Die Methacrylsäure wird von dem Wasser im Destillat durch fraktionierte Destillation abgetrennt.

Es ist ein besonderer Vorteil des erfindungsgemässen Verfahrens, dass nur leicht zugängliche Einsatzstoffe erforderlich sind. Der Rohstoff Isobutyraldehyd fällt bei der Oxosynthese zum Teil in erheblichen Mengen an, er entsteht aus Propylen und Synthesegas. Als weiterer Rohstoff ist nur noch Sauerstoff bzw. Luft erforderlich, z.T. über $HNO_3$ als $O_2$-Überträger. Der Alkohol, insbesondere Methanol, läuft im Kreis um. 1 kg Isobutyraldehyd ergibt über alle Stufen $\geqslant$ 0,75 kg Methacrylsäure.

Die Methacrylsäure wird z.T. direkt, zum grösseren Teil als Ester benötigt. Die Veresterung nach oder in Kombination mit der letzten Stufe ist auch möglich.

Alle Stufen sind kontinuierlich oder diskontinuierlich durchzuführen. Das beanspruchte Verfahren ist sehr umweltfreundlich. Man vermeidet das Arbeiten mit dem gefährlichen Cyanwasserstoff. Zudem fallen ausser Aceton und Alkylformiat keine nennenswerten Mengen an Nebenprodukten an. Man erhält die Methacrylsäure in einer hohen Reinheit, so dass sie oder deren Ester für die Polymerisation zu Polymethacrylaten direkt eingesetzt werden kann.

*Beispiele*

1.1 *Die Acetalbildung*

1.1.1 *Acetalisierung mit p-Toluolsulfonsäure*

Isobutyraldehyd und Methanol vereint man in stöchiometrischen Mengen bei 25° C in Gegenwart von 0,3 Gew.-% p-Toluolsulfonsäure, bezogen auf Ansatzmenge. Unter Erwärmung auf ca. 60° C stellt sich in wenigen Minuten das Gleichgewicht ein (60 bis 70% Umsatz). Die Säure neutralisiert man mit der berechneten Menge NaOH. Das Reaktionsprodukt wird mehrmals mit Wasser gewaschen, um nichtumgesetzes Methanol und Isobutyraldehyd abzutrennen. Das gewaschene Produkt wird destilliert. Das Acetal geht über bei 100 bis 104° C. Die im Waschwasser enthaltenen organischen Bestandteile werden durch Destillation zurückgewonnen.

1.1.2 *Acetalisierung mit Ionenaustauscher*

450 g/h eines Isobutyraldehyd/Methanol-Gemisches (50 Mol-% Isobutyraldehyd und 50 Mol-% Methanol) gibt man kontinuierlich in einen mit 250 ml eines sauren Ionenaustauschers (auf der Basis von vernetzten, sulfonierten Styrolpolymerisaten) gefüllten Rohrspiralenreaktor, Rohrdurchmesser i 10 mm, Rohrlänge 4 m. Die Reaktionstemperatur wird bei 100° C, der Betriebsdruck bei 5 bar gehalten. Der kontinuierlich anfallende Reaktionsaustrag enthält neben nichtumgsetztem Isobutyraldehyd und Methanol das gewünschte Acetal, Isobutenylmethylether und Reaktionswasser. Der Reaktionsaustrag wird wie im Beispiel 1.1.1 beschrieben aufgearbeitet.

1.2 *Die Acetalspaltung zum Isobutenylmethylether*

1.2.1 *Spaltung*

Isobutyraldehyddimethylacetal bringt man mit 0,5% p-Toluolsulfonsäure in einem Destillationskolben mit aufgesetzter Kolonne zum Sieden

(Sumpftemperatur 100° C). Durch die einsetzende Spaltung des Acetals zum Isobutenylmethylether und Methanol stellt sich in der Dampfphase eine Temperatur von 55 bis 57° C ein. Bei dieser Temperatur destilliert man das leichtsiedende Spaltproduktgemisch ab. Der Ether wird durch eine Wasserwäsche von Methanol befreit, getrocknet und destilliert. Der Ether siedet bei 75 bis 77° C. Das Methanol aus dem Wasser gewinnt man durch Andestillieren. Die Ausbeute der beiden ersten Stufen hängt ab von der Intensität der Methanol-Isobutyraldehyd-Rückgewinnung aus dem Wasser und liegt zwischen 90 bis 95 Mol-%. Ohne die Wasseraufarbeitung erzielt man eine Ausbeute von 40%.

1.2.2 *Spaltung mit Zusatz von Paraffinöl*

Isobutyraldehyddimethylacetal erhitzt man mit 0,5% p-Toluolsulfonsäure und mit Paraffinöl in einer Blase mit aufgesetzter Kolonne (Gewichtsverhältnis Acetal: Paraffinöl = 1 : 0,4, Sumpftemperatur 100 bis 120° C). Man erhitzt und destilliert das Gemisch Ether und Methanol kontinuierlich ab.

Die Reingewinnung des Ethers und die Rückgewinnung des Methanols geschieht wie im Beispiel 1.2.1 beschrieben.

1.3 *Oxidation des Isobutenylmethylethers unter Bildung von Isobutenyloxydmethylether*

1.3.1 *Ether-Oxidation mit reinem Sauerstoff*

In einer Oxidationsapparatur, deren Reaktionsteil aus einem Umlaufreaktor mit einem Reaktionsrohr von 40 mm Innendurchmesser, 2000 mm Höhe und einem temperierbaren Produktumlauf besteht, begast man 2660 g Isobutenylmethylether, die 200 ppm KOH enthalten, bei 34° C mit 50 l/h reinem, molekularem Sauerstoff. Der Sauerstoff gelangt über eine Fritte unten in das Reaktionsrohr. Das Abgas enthält nichtumgesetzten Sauerstoff, aber praktisch kein CO oder $CO_2$. Aus dem Reaktor ausgetragene Leichtsieder, wie z.B. Aceton und Methylformiat, kondensiert man in einem Kühlsystem. Bei einem Etherumsatz von ~ 40% steigert man zur Erhaltung des fast vollständigen Sauerstoffumsatzes die Reaktionstemperatur gleitend auf 55° C und reduziert den Sauerstoffzugang stetig von 50 auf 10 l/h.

Folgende Werte für Umsatz und Ausbeute werden erzielt

| Umsatz/ Ether (%) | Ausbeute (Mol-%) | | | |
|---|---|---|---|---|
| | Epoxid | Methyl-formiat | Aceton | α-Hydroxyisobutyr-aldehyddimethylacetal |
| 67 | 75 | 1 | 10 | 3 |
| 88 | 69 | 1,5 | 18 | 5 |

Den Reaktoraustrag versetzt man nun mit 0,3 Gew.-% KOH – in wenig Methanol gelöst – und fraktioniert bei Unterdruck. Bei 300 mbar werden der nichtumgesetzte Ether, Aceton und Methylformid abdestilliert; das Epoxid siedet bei 150 mbar und 45 bis 46° C. Die Reinheit beträgt 95% Epoxid.

1.3.2 *Etheroxidation mit Luft*

2750 g Isobutenylmethylether werden mit 500 ppm Kaliumhydroxid in der oben beschriebenen Oxidationsapparatur vorgelegt. Bei einer Reaktionstemperatur von 50° C wird mit 80 l/h Luft begast. Die Luft gelangt über eine Fritte von unten in das Reaktionsrohr. Mit dem Abgas aus dem Reaktor ausgetragene Leichtsieder, wie z.B. nichtumgesetzter Ether, Aceton und Methylformiat, werden in einem Kühlsystem kondensiert und in den Reaktor zurückgefahren. Bei einem Etherumsatz von ~ 40% steigert man zur Erhaltung des fast vollständigen Sauerstoffumsatzes die Reaktionstemperatur gleitend auf 60° C und reduziert den Luftzugang, ebenfalls gleitend, auf 20 l/h. Der Reaktionsaustrag wird wie im Beispiel 1.3.1 beschrieben aufgearbeitet. Die Ausbeuten entsprechen denen der Sauerstoffoxidation.

1.4 *Wasseranlagerung an das Epoxid unter Bildung von α-Hydroxyisobutyraldehyd*

1.4.1 *Hydrolyse von destilliertem Epoxid*

Die Epoxidfraktion wird nach Abzug des Ethers und der angefallenen Leichtsieder zusammen mit dem danach überdestillierenden α-Hydroxyisobutyraldehyddimethylacetal hydrolysiert. Hierzu legt man die berechnete Menge Wasser mit 10% Überschuss im Kolben vor und versetzt unter Rühren langsam in einer Zeit von ca. 60 min mit dem Epoxid. Die Reaktionstemperatur hält man durch Kühlung auf 66° C. Nach Beendigung der Wärmebildung destilliert man den Kolbeninhalt vorsichtig an und stellt mit $HNO_3$ auf $pH_3$ ein. Dabei entfernt man das Methanol. Den verbleibenden Rückstand setzt man direkt in die Salpetersäureoxidation ein.

1.4.2 *Hydrolyse von Rohepoxid*

320 g Wasser werden in einem Kolben vorgelegt und unter Rühren mit 1700 g Rohepoxid, wie z.B. der Oxidationsaustrag in Beispiel 1.3.2, versetzt (Dauer 30 min). Die Reaktionstemperatur wird dabei durch leichte Kühlung und durch einsetzenden Methanol- und Leichtsiederrücklauf bei 66° C gehalten. Nach Beendigung der Wärmebildung destilliert man den Reaktionsaustrag an, um den nichtumgesetzten Ether, Methanol und aus dem Einsatzprodukt vorhandenen Leichtsieder zu entfernen.

1.5 *Die Salpetersäureoxidation*

1.5.1. *Oxidation mit 100%iger Salpetersäure*

500 g Rückstand aus der Hydrolyse mit 3,6 mol Aldehyd und 0,9 mol α-Hydroxyisobutyraldehyddimethylacetal erwärmt man in einem Kolben auf 70° C und versetzt mit 5,8 mol 100%iger Salpetersäure (rauchende Säure). Durch Kühlung hält man die Temperatur auf 80° C. Die entweichenden Gase sind im wesentlichen NO, $NO_2$ und je nach Methanolgehalt Ester, vorrangig Salpetrigsäureester. $N_2$ und $N_2O$ treten nur in Spuren auf. Nach Abschluss der Wärme- und Gasentwicklung wird das flüssige Produkt im Vakuum destilliert. Bei 200 bis 15 mbar wird ein Vorlauf abgezogen, der hauptsächlich aus Wasser und $HNO_3$ besteht. Bei 0,5 bis 1 mbar siedet die weisse kristallisierende α-Hydroxyisobuttersäure bei einer Kopftemperatur von ca. 90° C. In einer einfachen Laborbrücke geht etwa 80% der α-Hydroxyisobuttersäure als 95%ige Säure über. Sie hat einen Schmelzpunkt von 75° C.

Die Ausbeute an α-Hydroxyisobuttersäure über die Schritte Epoxid-Hydrolyse—Salpetersäureoxidation beträgt 90 bis 95 Mol-%.

1.5.2. *Oxidation mit 65%iger Salpetersäure*

980 g konzentrierte 65%ige Salpetersäure werden in einem Kolben unter Rühren bei 20° C vorgelegt. Dann gibt man innerhalb von 45 min kontinuierlich 500 g Roh-α-hydroxyisobutyraldehyd – 4,4 mol Aldehyd, 0,7 mol α-Hydroxyisobutyraldehyddimethylacetal mit 3,3 mol Wasser – hinzu. Es setzt sofort Wärme- und Gasentwicklung ein. Die Temperatur wird durch Kühlung bei etwa 40° C gehalten. Nach Abschluss der Aldehydzugabe wird der Ansatz zur Nachreaktion ca. 30 min bei 60 bis 70° C gehalten und anschliessend wie im Beispiel 1.5.1 beschrieben aufgearbeitet. Die Ausbeute an α-Hydroxyisobuttersäure beträgt 4,85 mol.

1.6 *Wasserabspaltung aus α-Hydroxyisobuttersäure zur Methacrylsäure*

350 g α-Hydroxyisobuttersäure (96,5%), 6 g Natriumhydroxid und 0,6 g Hydrochinonmethylether werden in einen Destillationskolben mit aufgesetzter Kolonne gegeben und zum Sieden auf ca. 200° C erhitzt, währenddessen in den Destillationskolben Luft mit einer Geschwindigkeit von 50 ml/min eingeführt wird. Die bei 92 bis 130° C überdestillierenden Reaktionsprodukte enthalten Methacrylsäure in einer Ausbeute von 95%. Die Methacrylsäure wird von dem mitanfallenden Wasser durch fraktionierte Destillation abgetrennt. Das Natriumhydroxid setzt sich mit der α-Hydroxyisobuttersäure zu dem Natriumsalz dieser Säure um, das als Dehydratisierungskatalysator wirkt.

**Patentansprüche**

1. Verfahren zur Herstellung von Methacrylsäure aus Isobutyraldehyd über mehrere Stufen, dadurch gekennzeichnet, dass man
1. in einer ersten Stufe in bekannter Weise Isobutyraldehyd acetalisiert,
2. in einer zweiten Stufe in bekannter Weise das Acetal zum Isobutenylether und Alkohol spaltet,
3. den Isobutenylether mit molekularem Sauerstoff oder einem sauerstoffhaltigen Gasgemisch in Gegenwart von 50 bis 500 ppm einer Lauge bei Temperaturen von 30 bis 70° C zum Epoxid oxidiert,
4. das Epoxid in bekannter Weise zum α-Hydroxyisobutyraldehyd hydrolysiert,
5. diesen anschliessend mit 1,2 bis 2 mol rauchender oder konzentrierter Salpetersäure pro Mol Aldehyd bei Temperaturen von 20 bis 110° C zur α-Hydroxyisobuttersäure oxidiert, und
6. aus der α-Hydroxyisobuttersäure in bekannter Weise durch Wasserabspaltung Methacrylsäure gewinnt.

2. Verfahren nach Anspruch 1, dadurch gekennzeichnet, dass man in der Stufe 3 als Lauge KOH oder NaOH einsetzt.

3. Verfahren nach den Ansprüchen 1 und 2, dadurch gekennzeichnet, dass man die Oxidation des Isobutenylethers bei Temperaturen von 30 bis 50° C mit einer Sauerstoffmenge von 15 bis 20 l/kg Isobutenylether beginnt und in laufender Anpassung an die umsatzabhängige Reaktionsgeschwindigkeit nach einem Umsatz von 80 bis 90% bei 55 bis 70° C mit einer Sauerstoffmenge von 5 bis 10 l/kg Isobutenylether zu Ende führt.

**Claims**

1. A multistage process for the production of methacrylic acid from isobutyraldehyde, characterised in that:
(1) isobutyraldehyde is acetalised in a known manner in a first stage;
(2) the acetal is cleaved in a known manner in a second stage to form isobutenyl ether and alcohol,
(3) the isobutenyl ether is oxidised to epoxide, at a temperature of 30 to 70° C, using molecular oxygen or a gas mixture containing oxygen in the presence of 50 to 500 ppm of an alkali;
(4) the epoxide is hydrolysed in a known manner to α-hydroxyisobutyraldehyde;
(5) the latter is then oxidised to α-hydroxyisobutyric acid, at a temperature of 20 to 110° C, with 1.2 to 2 mol of fuming or concentrated nitric acid per mol of aldehyde, and
(6) methacrylic acid is obtained in a known manner from the α-hydroxy-isobutyric acid by splitting off water.

2. A process according to Claim 1, characterised in that KOH or NaOH is used as the alkali in the third stage.

3. A process according to Claims 1 and 2, characterised in that the oxidation of the isobutenyl ether is begun at a temperature of 30 to 50° C, with an oxygen concentration of 15 to 20 l/kg of isobutenyl ether and with continual adjustment to the conversion-dependent reaction rate, and comes to an end after a conversion of 80 to 90%, at 55 to 70° C, and an oxygen concentration of 5 to 10 l/kg of isobutenyl ether.

**Revendications**

1. Procédé de préparation d'acide méthacrylique en partant d'isobutyraldéhyde et en passant par plusieurs étapes, caractérisé par le fait:

1) qu'en une première étape, de façon connue, on acétalise l'isobutyraldéhyde;
2) qu'en une deuxième étape, de façon connue, on décompose l'acétal en éther isobuténylique et en alcool;
3) que l'on oxyde l'éther isobuténylique en époxyde au moyen d'oxygène moléculaire ou d'un mélange gazeux contenant de l'oxygène, en présence de 50 à 500 ppm d'une lessive, à des températures de 30 à 70° C;
4) que l'on hydrolyse l'époxyde de façon connue en α-hydroxyisobutyraldéhyde;
5) qu'ensuite on oxyde celui-ci en acide α-hydroxyisobutyrique avec 1,2 à 2 mol d'acide nitrique fumant ou concentré par mole d'aldéhyde, à des températures de 20 à 110° C, et
6) qu'en partant de l'acide α-hydroxyisobutyrique, on obtient l'acide méthacrylique, de façon connue, par élimination d'eau.

2. Procédé selon la revendication 1, caractérisé par le fait qu'à l'étape 3 on utilise comme lessive KOH ou NaOH.

3. Procédé selon les revendications 1 et 2, caractérisé par le fait que l'on commence l'oxydation de l'éther isobuténylique à des températures de 30 à 50° C, avec une quantité d'oxygène de 15 à 20 l/kg d'éther isobuténylique, et que, avec adaptation continuelle à la vitesse de réaction, qui dépend de la conversion, après une conversion de 80 à 90%, on la conduit à l'achèvement entre 55 et 70° C avec une quantité d'oxygène de 5 à 10 l/kg d'éther isobuténylique.